(19) Europäisches Patentamt
European Patent Office
Office européen des brevets

(11) EP 4 421 087 A1

(12) EUROPEAN PATENT APPLICATION
published in accordance with Art. 153(4) EPC

(43) Date of publication:
**28.08.2024 Bulletin 2024/35**

(21) Application number: **22894680.2**

(22) Date of filing: **09.11.2022**

(51) International Patent Classification (IPC):
***C07K 14/605*** *(2006.01)* ***A61K 38/26*** *(2006.01)*
***A61P 1/00*** *(2006.01)*

(52) Cooperative Patent Classification (CPC):
**A61K 38/26; A61P 1/00; C07K 14/605**

(86) International application number:
**PCT/CN2022/130781**

(87) International publication number:
**WO 2023/088143 (25.05.2023 Gazette 2023/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.11.2021 CN 202111400591**
**08.06.2022 CN 202210648263**

(71) Applicant: **Soter Biopharma Pte. Ltd.**
**Singapore 179803 (SG)**

(72) Inventors:
- **PAN, Zhixiang**
  **Shanghai 200131 (CN)**
- **HE, Haiying**
  **Shanghai 200131 (CN)**
- **JIANG, Zhigan**
  **Shanghai 200131 (CN)**
- **CHEN, Shuhui**
  **Shanghai 200131 (CN)**

(74) Representative: **Cabinet Becker et Associés**
**25, rue Louis le Grand**
**75002 Paris (FR)**

Remarks:
The complete document including Reference Table(s) and the Sequence Listing(s) can be downloaded from the EPO website

# (54) STAPLE-CONTAINING POLYPEPTIDES AND APPLICATION THEREOF

(57) A series of staple-containing polypeptides and an application thereof, and specifically disclosed are polypeptides having sequences as shown in formulas (I-1)-(I-5).

EP 4 421 087 A1

## Description

**The present disclosure claims the priority of:**

CN2021114005918, filed on November 19, 2021;
CN2022106482638, filed on June 8, 2022.

## TECHNICAL FIELD

The present disclosure relates to a series of staple-containing polypeptides and use thereof. Specifically, the present disclosure discloses the polypeptides with sequences represented by formulas (1-1) to (I-5).

## BACKGROUND

Overweight and obesity are serious health problems facing all mankind. They are often accompanied by other diseases such as coronary artery disease, hypertension, type 2 diabetes, nonalcoholic fatty liver disease, kidney disease and certain cancers. The World Health Organization (WHO) defines obesity as one of the top ten chronic diseases. Obesity, along with hypertension, hyperlipidemia and hyperglycemia are known as the "Quartet of Death" and may become the number one killer in the 21st century. Data from WHO show that the prevalence of obesity in China was approximately 6.2% in 2016. In addition, the Lancet published a survey report of worldwide adult body weight in 2016. The survey found that the population of worldwide obese adults has exceeded that of healthy adults, and China has surpassed the United States to become the country with the largest obese population in the world. The number of people suffering from diabetes, hypertension, cardiovascular disease and other diseases caused by overweight and obesity is increasing year by year, and ages of these people are getting younger.

Glucagon (GCG) is a hormone secreted by pancreas and binding to the glucagon receptor (GCGR) to produce physiological functions. Glucagon promotes the rise of blood sugar by increasing gluconeogenesis and glycogenolysis. In addition, GCG can also reduce the synthesis of fatty acid in liver adipose tissue and promote fat decomposition. Glucagon-like peptide 1 (GLP-1) is a hormone secreted by intestinal L cells. It can reduce body weight by suppressing appetite and reducing food intake, as well as increasing energy consumption and promoting the thermogenesis of brown adipose tissue. On the basis of maintaining the efficacy of a GLP-1 agonist, the introduction of GCG activity will have the medicinal effects of: helping to further promote the secretion of insulin from pancreatic β cells; promoting the metabolism of brown adipose tissue; enhancing the β-oxidation of the fatty acids in liver and reducing the generation of lipids and cholesterol; improving the survival rate of cardiomyocytes; accelerating the lipid metabolism of white adipose tissue and reducing the fat content. The GLP-1/GCG dual-target synergy is likely to have better effect in improving blood sugar and weight loss than a single-target action. Therefore, the research on the GLP-1/GCG dual-target drugs in the treatment of obesity and related diseases is of great significance.

## SUMMARY

The present disclosure provides a polypeptide with a sequence represented by the following formulas,

His-Aib-Gln-Gly-Thr-Phe-Thr-Ser-Asp-Tyr-Ser-Lys-Tyr-Leu-Asp-Glu-$^{1}$Lys-Lys-Ala-$^{1}$Lys-Glu-Phe-Val-Glu-Trp-Leu-Leu-Glu-Gly-Gly-Pro-Ser-Ser-$X_2$ (I-1)

His-Aib-Gln-Gly-Thr-Phe-Thr-Ser-Asp-Tyr-Ser-Lys-Tyr-Leu-Asp-Glu-$^{1}$Lys-Lys-Ala-Lys-$^{1}$Lys-Phe-Val-Glu-Trp-Leu-Leu-Glu-Gly-Gly-Pro-Ser-Ser-$X_2$ (I-2)

His-Aib-Gln-Gly-Thr-Phe-Thr-Ser-Asp-Tyr-Ser-Lys-Tyr-Leu-Asp-Glu-Lys-$^{1}$Lys-Ala-Lys-$^{1}$Lys-Phe-Val-Glu-Trp-Leu-Leu-Glu-Gly-Gly-Pro-Ser-Ser-$X_2$ (I-3)

His-Aib-Gln-Gly-Thr-Phe-Thr-Ser-Asp-Tyr-Ser-Lys-Tyr-Leu-Asp-Glu-Lys-Lys-Ala-$^{1}$Lys-Glu-Phe-Val-$^{1}$Lys-Trp-Leu-Leu-Glu-Gly-Gly-Pro-Ser-Ser-$X_2$ (I-4)

His-Aib-Gln-Gly-Thr-Phe-Thr-Ser-Asp-Tyr-Ser-Lys-Tyr-Leu-Asp-Glu-Lys-Lys-Ala-Lys-Glu-Phe-Val-$^{1}$Lys-Trp-Leu-Leu-$^{1}$Lys-Gly-Gly-Pro-Ser-Ser-$X_2$ (I-5)

wherein

the structure of Aib is

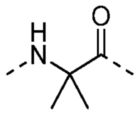
;

$X_2$ is selected from

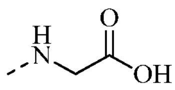
and
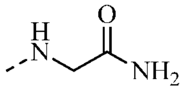
;

$^{1}$Lys represents a modified lysine, and the modification is that the amino groups on the two lysine side chains are connected to

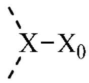
;

X is selected from

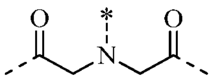
and
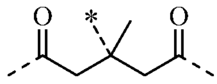
,

wherein "*" indicates the position connected to $X_0$;
$X_0$ is

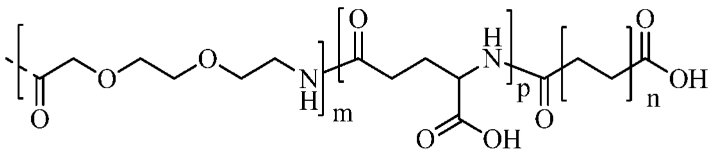
;

m is selected from 2 and 3;
n is selected from 8, 9 and 10;
p is selected from 1 and 2.

In some embodiments of the present disclosure, the above-mentioned m is 2, and other variables are as defined in the present disclosure.
In some embodiments of the present disclosure, the above-mentioned n is 9, and other variables are as defined in the present disclosure.
In some embodiments of the present disclosure, the above-mentioned p is 1, and other variables are as defined in the present disclosure.
In some embodiments of the present disclosure, the above-mentioned $X_2$ is

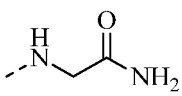
,

and other variables are as defined in the present disclosure.
In some embodiments of the present disclosure, the above-mentioned $X_0$ is

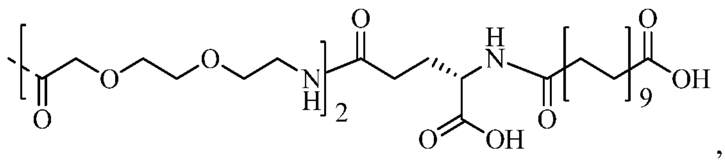

,

and other variables are as defined in the present disclosure.

In some embodiments of the present disclosure, the above-mentioned structural unit

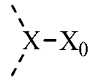 is 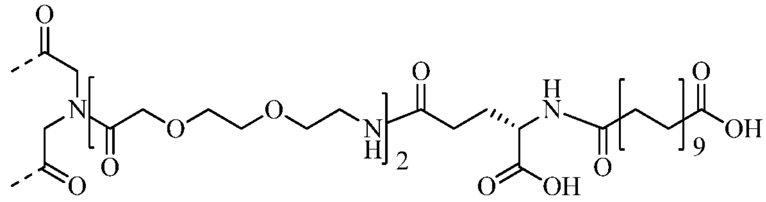

,

and other variables are as defined in the present disclosure.

The present disclosure also includes some embodiments that are obtained by combining any of the above-mentioned variables.

The present disclosure also provides a polypeptide represented by the following formulas,

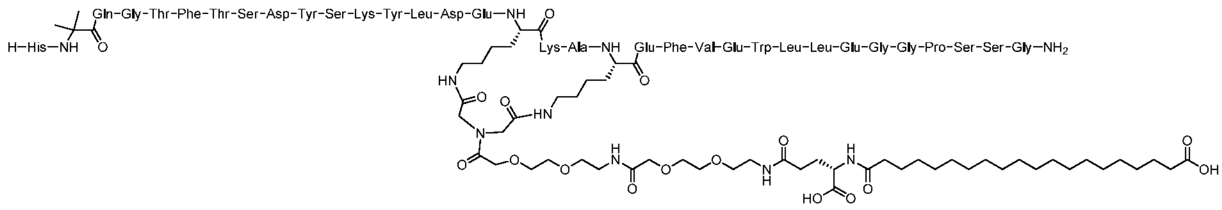

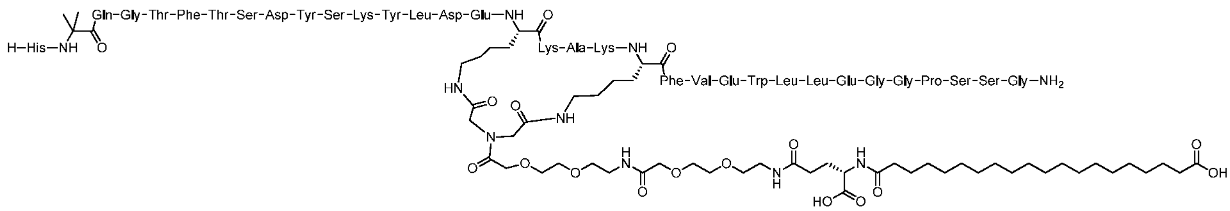

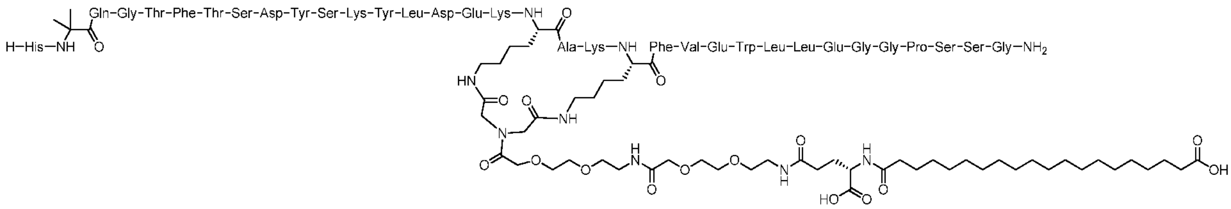

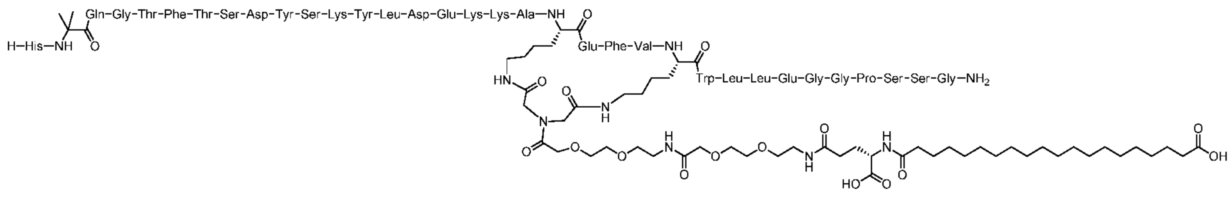

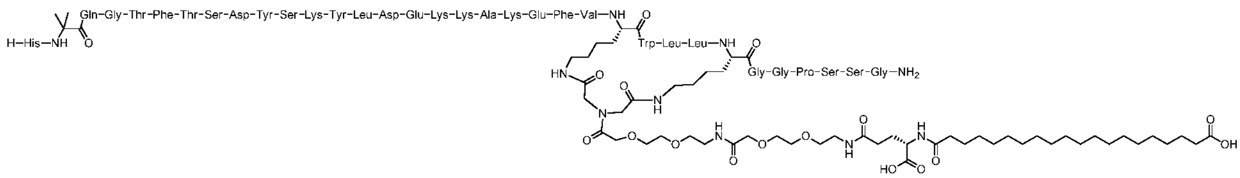

.

The present disclosure also provides use of an above-mentioned polypeptide compound in the manufacture of a medicament for the treatment of a GLP-1R/GCGR-related disease.

In some embodiments of the present disclosure, the GLP-1R/GCGR-related disease is selected from obesity and nonalcoholic steatohepatitis (NASH).

## Technical effect

The compounds of the present disclosure have strong agonistic activity on GLP-1R/GCGR; the compounds of the present disclosure exhibit excellent weight-loss efficacy in DIO mice; the compounds of the present disclosure exhibit excellent NASH-improving efficacy in STZ-NASH mice; the compounds of the present disclosure have extremely high plasma protein binding and excellent plasma stability; the compounds of the present disclosure have excellent pharmacokinetic properties.

## Definition and term

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" means a salt of compounds disclosed herein that is prepared by reacting the compound having a specific substituent disclosed herein with a relatively non-toxic acid or base. When compounds disclosed herein contain a relatively acidic functional group, a base addition salt can be obtained by bringing the compound into contact with a sufficient amount of base in a pure solution or a suitable inert solvent. When compounds disclosed herein contain a relatively basic functional group, an acid addition salt can be obtained by bringing the compound into contact with a sufficient amount of acid in a pure solution or a suitable inert solvent. Some specific compounds disclosed herein contain both basic and acidic functional groups and can be converted to any base or acid addition salt.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

"Amino acid" refers to naturally occurring and synthetic amino acids, as well as amino acid analogs and amino acid mimetics that perform a similar function to the naturally occurring amino acids. The naturally occurring amino acids are those encoded by a genetic code, as well as those that are later modified, such as hydroxyproline, $\gamma$-carboxyglutamic acid, and O-phosphoserine. The amino acid analog refers to a compound that has the same basic chemical structure (e.g., an $\alpha$ carbon bonded to a hydrogen, a carboxyl group, an amino group, or an R group) as a naturally occurring amino acid, such as homoserine, norleucine, methionine sulfoxide, and methionine methylsulfonium. Such analog may have a modified R group (e.g., norleucine) or a modified peptide backbone, but retains the same basic chemical structure as the naturally occurring amino acid. The amino acid mimetic refers to a chemical compound that has a structure different from the general chemical structure of an amino acid but performs a similar function to a naturally occurring amino acid.

A or Ala disclosed herein represents alanine, with a structure of

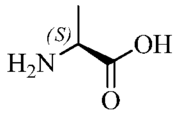;

R or Arg represents arginine, with a structure of

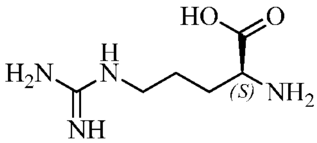;

N or Asn represents asparagine, with a structure of

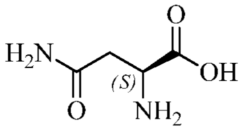;

D or Asp represents aspartic acid, with a structure of

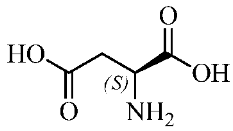;

C or Cys represents cysteine, with a structure of

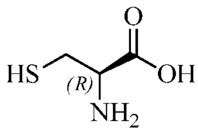;

Q or Gln represents glutamine, with a structure of

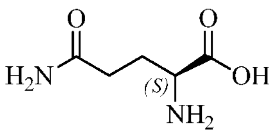;

E or Glu represents glutamic acid, with a structure of

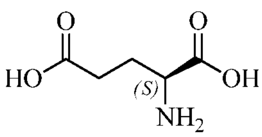;

G or Gly represents glycine, with a structure of

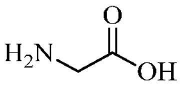;

H or His represents histidine, with a structure of

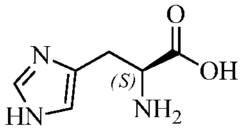;

I or Ile represents isoleucine, with a structure of

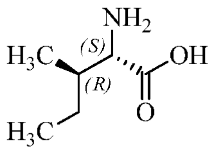;

L or Leu represents leucine, with a structure of

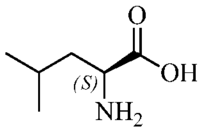;

K or Lys represents lysine, with a structure of

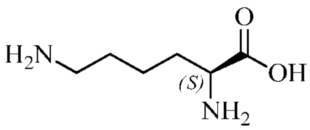;

M or Met represents methionine, with a structure of

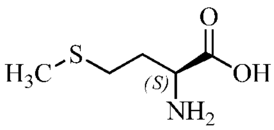;

F or Phe represents phenylalanine, with a structure of

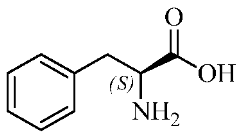;

P or Pro represents proline, with a structure of

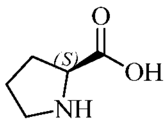

;

S or Ser represents serine, with a structure of

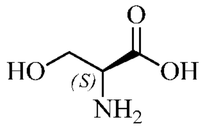

;

T or Thr represents threonine, with a structure of

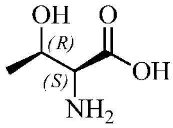

;

W or Trp represents tryptophan, with a structure of

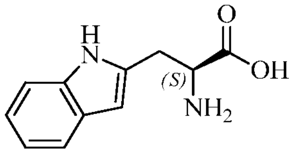

;

Y or Tyr represents tyrosine, with a structure of

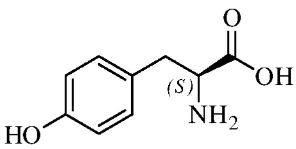

;

V or Val represents valine, with a structure of

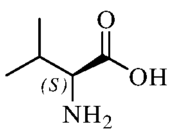

.

The term "treatment" includes inhibiting, slowing, stopping or reversing the existing symptoms or the progression or severity of a patient's condition.

Unless otherwise specified, the term "isomer" is intended to include geometric isomers, cis- or trans- isomers, stereoisomers, enantiomers, optical isomers, diastereomers, and tautomers.

Compounds disclosed herein may be present in a specific geometric or stereoisomeric form. The present disclosure contemplates all such compounds, including cis and trans isomers, (-)- and (+)-enantiomers, (R)- and (*S*)-enantiomers, diastereoisomers, (D)-isomers, (*L*)-isomers, and a racemic mixture and other mixtures, for example, a mixture enriched in enantiomer or diastereoisomer, all of which are encompassed within the scope disclosed herein. The substituent such as alkyl may have an additional asymmetric carbon atom. All these isomers and mixtures thereof are encompassed within the scope disclosed herein.

Unless otherwise specified, the term "enantiomer" or "optical isomer" means stereoisomers that are in a mirrored

relationship with each other.

Unless otherwise specified, the term "cis-trans isomer" or "geometric isomer" is produced by the inability of a double bond or a single bond between ring-forming carbon atoms to rotate freely.

Unless otherwise specified, the term "diastereomer" means a stereoisomer in which two or more chiral centers of are contained in a molecule and is in a non-mirrored relationship between molecules.

Unless otherwise specified, "(+)" means dextroisomer, "(-)" means levoisomer, and "(±)" means racemate.

Unless otherwise specified, a wedged solid bond

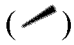

and a wedged dashed bond

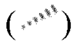

indicate the absolute configuration of a stereocenter; a straight solid bond

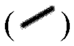

and a straight dashed bond

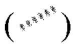

indicate the relative configuration of a stereocenter; a wavy line

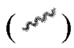

indicates a wedged solid bond

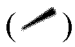

or a wedged dashed bond

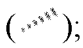;

or a wavy line

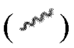

indicates a straight solid bond

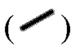

or a straight dashed bond

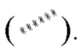.

Unless otherwise specified, the term "enriched in one isomer", "isomer enriched", "enriched in one enantiomer" or "enantiomeric enriched" means that the content of one isomer or enantiomer is less than 100%, and the content of

the isomer or enantiomer is 60% or more, or 70% or more, or 80% or more, or 90% or more, or 95% or more, or 96% or more, or 97% or more, or 98% or more, or 99% or more, or 99.5% or more, or 99.6% or more, or 99.7% or more, or 99.8% or more, or 99.9% or more.

Unless otherwise specified, the term "isomer excess" or "enantiomeric excess" means the difference between the relative percentages of two isomers or two enantiomers. For example, if one isomer or enantiomer is present in an amount of 90% and the other isomer or enantiomer is present in an amount of 10%, the isomer or enantiomeric excess (ee value) is 80%.

Optically active (R)- and (S)-isomer, or D and L isomer can be prepared using chiral synthesis or chiral reagents or other conventional techniques. If one kind of enantiomer of certain compound disclosed herein is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to afford the pure enantiomer. In addition, the enantiomer and the diastereoisomer are generally isolated through chromatography which uses a chiral stationary phase and optionally combines with a chemical derivative method (for example, carbamate generated from amine).

Compounds disclosed herein may contain an unnatural proportion of atomic isotopes at one or more of the atoms that make up the compounds. For example, a compound may be labeled with a radioisotope such as tritium ($^{3}H$), iodine-125 ($^{125}I$) or C-14($^{14}C$). For another example, hydrogen can be replaced by heavy hydrogen to form a deuterated drug. The bond between deuterium and carbon is stronger than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

When an enumerated linking group does not indicate its linking direction, its linking direction is arbitrary. For example, when the linking group L in

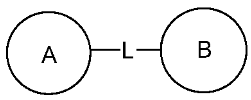

is -M-W-, the -M-W- can be linked to the ring A and the ring B in the same direction as the reading order from left to right to constitute

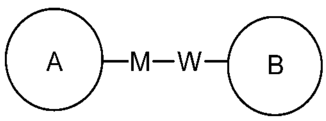
,

or can be linked to the ring A and the ring B in the reverse direction as the reading order from left to right to constitute

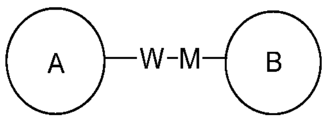
.

A combination of the linking groups, substituents and/or variants thereof is allowed only when such combination can result in a stable compound.

Unless otherwise specified, when a group has one or more connectable sites, any one or more sites of the group can be connected to other groups through chemical bonds. When the connection position of the chemical bond is variable, and there is H atom(s) at a connectable site(s), with the connection of the chemical bond, the number of H atom(s) at this site will correspondingly decrease as the number of the connected chemical bond increases, and the group will become a group of corresponding valence. The chemical bond between the site and other groups can be represented by a straight solid bond

(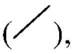),

a straight dashed bond

(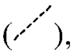),

, or a wavy line

(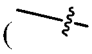).

For example, the straight solid bond in $-OCH_3$ indicates that the group is connected to other groups through the oxygen atom in the group; the straight dashed bond in

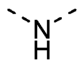

indicates that the group is connected to other groups through two ends of the nitrogen atom in the group; the wavy line in

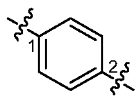

indicates that the group is connected to other groups through the 1- and 2-carbon atoms in the phenyl group.

Unless otherwise specified, the term "$C_{1-3}$ alkyl" is used to indicate a linear or branched saturated hydrocarbon group consisting of 1 to 3 carbon atoms. The $C_{1-3}$ alkyl group includes $C_{1-2}$ and $C_{2-3}$ alkyl groups and the like. It may be monovalent (e.g., methyl), divalent (e.g., methylene) or multivalent (e.g., methenyl). Examples of $C_{1-3}$ alkyl groups include, but are not limited to, methyl (Me), ethyl (Et), propyl (including n-propyl and isopropyl), and the like.

The structures of compounds disclosed herein can be confirmed by conventional methods well known to those skilled in the art. If the present disclosure relates to an absolute configuration of a compound, the absolute configuration can be confirmed by conventional techniques in the art, such as single crystal X-Ray diffraction (SXRD). In a single crystal X-Ray diffraction (SXRD), the diffraction intensity data of a cultivated single crystal are collected using a Bruker D8 venture diffractometer with a light source of CuK$\alpha$ radiation in a scanning mode of $\varphi/\omega$ scan; after collecting the relevant data, the crystal structure is further analyzed by the direct method (Shelxs97) to confirm the absolute configuration.

Compounds disclosed herein can be prepared by a variety of synthetic methods well known to those skilled in the art, including the following enumerated embodiment, the embodiment formed by the following enumerated embodiment in combination with other chemical synthesis methods, and equivalents well known to those skilled in the art. Alternative embodiments include, but are not limited to examples disclosed herein.

Solvents used in the present disclosure are commercially available.

The following abbreviations are used in the present disclosure: aq represents aqueous; eq represents equivalent or equivalence; DCM represents dichloromethane; PE represents petroleum ether; DMSO represents dimethyl sulfoxide; MeOH represents methanol; Boc represents tert-butoxycarbonyl, which is an amine protecting group; Dde represents (4,4-dimethyl-2,6-dioxocyclohexylidene)ethyl, which is a side chain protecting group for amino acids; r.t. represents room temperature; O/N represents overnight; THF represents tetrahydrofuran; $Boc_2O$ represents di-tert-butyl dicarbonate; TFA represents trifluoroacetic acid; DIEA represents diisopropylethylamine; DMF represents N,N-dimethylformamide; HBTU represents O-(benzotriazol-1-yl)-N,N,N',N'-tetramethyluronium hexafluorophosphate; HOBT represents 1-hydroxybenzotriazole; HOAT represents 1-hydroxy-7-azabenzotriazole; DIC represents N,N'-diisopropylcarbodiimide; DBU represents 1,8-diazabicyclo[5.4.0]undec-7-ene; $PhSiH_3$ represents phenylsilane; $Pd(PPh_3)_4$ represents tetrakis(triphenylphosphine)palladium; AEEA represents 2-(2-(2-aminoethoxy)ethoxy)acetic acid; DIEA represents diisopropylethylamine.

Compounds are named according to general naming principles in the art or by ChemDraw® software, and commercially available compounds are named with their vendor directory names.

## DETAILED DESCRIPTION

The present disclosure is described in detail below by means of examples. However, it is not intended that these examples have any disadvantageous limitations to the present disclosure. The present disclosure has been described in detail herein, and embodiments are also disclosed herein. It will be apparent to those skilled in the art that various changes and modifications may be made to the embodiments disclosed herein without departing from the spirit and scope disclosed herein.

### Example 1

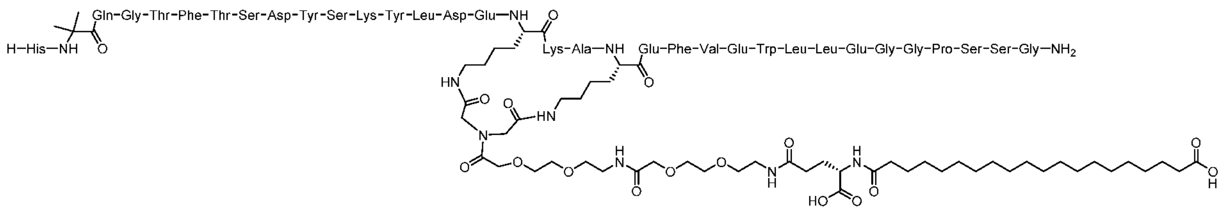

**WX001**

Synthesis of intermediate 1

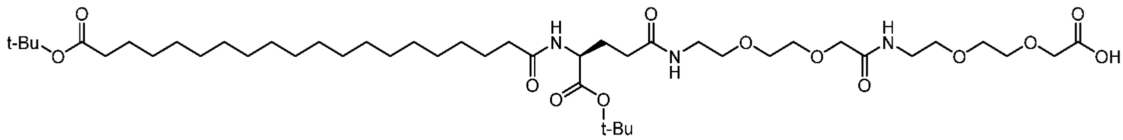

Intermediate 1

1. **Attachment of resin to intermediate 1**

1.1 5.0 g of 2-chlorotrityl chloride resin (2-CTC Resin, degree of substitution S=1.00 mmol/g) and 1.93 g of Fmoc-AEEA-OH were weighed and added to a reaction column, and DCM (40 mL) was further added. Then, DIEA (3.5 mL) was added to the reaction column and the system was blown with nitrogen for 2 h. Then, MeOH (5 mL) was added to the reaction column and the system was further blown with nitrogen for 30 min. The reaction column was drained until no liquid flowed out. DMF (50 mL) was added to wash 5 times, for 1 minute each time, and the reaction column was drained until no liquid flowed out.

1.2 20% piperidine/DMF (100 mL) was added to the reaction column and the system was blown with nitrogen for 20 minutes. The reaction column was drained until no liquid flowed out. DMF (100 mL) was added to wash 5 times, for 1 min each time. The reaction column was drained until no liquid flowed out. The resin was tested with ninhydrin and showed blue.

**2. Coupling of amino acids**

**2.1 Coupling of Fmoc-AEEA-OH**

1. Fmoc-AEEA-OH (3.0 eq) was weighed and added to the above resin. DIEA (6.0 eq) was added, and DMF (30 mL) was further added to the reaction column. The reaction column was blown with nitrogen. After the amino acid was dissolved, HBTU (2.85 eq) was added. The nitrogen was adjusted so that the resin was evenly blown.
2. The mixture was reacted in an environment of 25 °C for 20 minutes. The resin was tested with ninhydrin and was colorless and transparent.

3. The reaction solution was aspirated. The reaction column was washed 5 times with DMF, for 1 min each time, and drained until no liquid flowed out.

**2.2 Coupling of Fmoc-Glu-OtBu**

1. 20% piperidine/DMF (100 mL) was added to the reaction column and the system was blown with nitrogen for 20 minutes. The reaction column was drained until no liquid flowed out. The resin was tested with ninhydrin and showed blue.
2. Fmoc-Glu-OtBu (3.0 eq) was weighed and added to the above resin. DIEA (6.0 eq) was added, and DMF (30 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF (100 mL) 5 times, for 1 min each time, and drained until no liquid flowed out.

**2.3 Coupling of 20-(tert-butoxy)-20-oxoicosanoic acid**

1. 20% piperidine/DMF (100 mL) was added to the reaction column and the system was blown with nitrogen for 20 minutes. The reaction column was drained until no liquid flowed out. DMF (100 mL) was added to wash 5 times, for 1 min each time. The reaction column was drained until no liquid flowed out. The resin was tested with ninhydrin and showed blue.
2. 20-(tert-butoxy)-20-oxoicosanoic acid (3.0 eq) was weighed and added to the above resin. DIEA (6.0 eq) was added, and DMF (30 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times, for 1 min each time, and drained until no liquid flowed out.

**3. Cleavage and drying of crude peptide**

3.1. The cleavage solution was prepared according to the following volume:

$$HFIP/DCM=20/80.$$

3.2. 100 mL of the prepared cleavage solution was poured into a reactor containing the dried peptide resin. The reactor was blown for 20 minutes. The mixture was filtered, and the filtrate was added into a flask. This operation was repeated twice. The cleavage solution collected twice was rotary evaporated to dryness to give 4.3 g of crude peptide.

**Synthesis of WX001**

**1. Attachment of resin**

1.1 1.43 g of 4-(2',4'-dimethoxyphenyl-Fmoc-aminomethyl)-phenoxyacetamido-methyldiphenylmethylamine resin (Rink Amide MBHA Resin, degree of substitution Sub=0.28 mmol/g) was weighed and added to a reaction column. Then, DCM (50 mL) was added to the reaction column and the system was blown with nitrogen for 2 h. The reaction column was drained until no liquid flowed out. DMF (50 mL) was added to wash 5 times, for 1 minute each time, and the reaction column was drained until no liquid flowed out.
1.2 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and

showed blue.

### 2. Coupling of amino acids

### 2.1 Coupling of Fmoc-Gly-OH

1. Fmoc-Gly-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
2. The mixture was reacted in an environment of 25 °C for 0.5 h. The resin was tested with ninhydrin and was colorless and transparent.
3. The reaction solution was aspirated. The reaction column was washed with DMF 5 times (50 mL each time), for 1 min each time, and drained until no liquid flowed out.

### 2.2 Coupling of Fmoc-Ser(tBu)-OH

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Ser(tBu)-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

### 2.3 Coupling of Fmoc-Ser(tBu)-OH

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Ser(tBu)-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times, for 1 min each time, and drained until no liquid flowed out.

### 2.4 Coupling of Fmoc-Pro-OH

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Pro-OH (3.0 eq) was weighed and added to the above resin. DIEA (6.0 eq) was added, and DMF (10 mL) was further added to the reaction column. The reaction column was blown with nitrogen. After the amino acid was

dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.5 Coupling of Fmoc-Gly-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with chloranil and showed blue.
2. Fmoc-Gly-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with chloranil and was colorless and transparent.
4. The reaction solution was aspirated. The reaction column was washed with DMF 5 times (50 mL each time), for 1 min each time, and drained until no liquid flowed out.

**2.6 Coupling of Fmoc-Gly-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Gly-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.7 Coupling of Fmoc-Glu(OtBu)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Glu(OtBu)-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.8 Coupling of Fmoc-Leu-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Leu-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.9 Coupling of Fmoc-Leu-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Leu-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.10 Coupling of Fmoc-Trp(Boc)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Trp(Boc)-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.11 Coupling of Fmoc-Glu(OtBu)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Glu(OtBu)-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for

1 min each time, and drained until no liquid flowed out.

### 2.12 Coupling of Fmoc-Val-OH

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Val-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

### 2.13 Coupling of Fmoc-Phe-OH

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Phe-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

### 2.14 Coupling of Fmoc-Glu(OtBu)-OH

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Glu(OtBu)-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

### 2.15 Coupling of Fmoc-Lys(Dde)-OH

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.

2. Fmoc-Lys(Dde)-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.16 Coupling of Fmoc-Ala-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Ala-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.17 Coupling of Fmoc-Lys(Boc)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Lys(Boc)-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.18 Coupling of Fmoc-Lys(Alloc)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Lys(Alloc)-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.19 Coupling of Fmoc-Glu(OtBu)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Glu(OtBu)-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.20 Coupling of Fmoc-Asp(OtBu)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Asp(OtBu)-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.21 Coupling of Fmoc-Leu-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Leu-OH (3.0 eq) was weighed and added to the above resin. DIEA (6.0 eq) was added, and DMF (10 mL) was further added to the reaction column. The reaction column was blown with nitrogen. After the amino acid was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.22 Coupling of Fmoc-Tyr(tBu)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Tyr(tBu)-OH (3.0 eq) was weighed and added to the above resin. DIEA (6.0 eq) was added, and DMF (10 mL) was further added to the reaction column. The reaction column was blown with nitrogen. After the amino acid

was dissolved, HBTU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.23 Coupling of Fmoc-Lys(Boc)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Lys(Boc)-OH (6.0 eq) was weighed and added to the above resin. DIEA (12.0 eq) was added, and DMF (10 mL) was further added to the reaction column. The reaction column was blown with nitrogen. After the amino acid was dissolved, HATU (5.70 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.24 Coupling of Fmoc-Ser(tBu)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Ser(tBu)-OH (6.0 eq) was weighed and added to the above resin. DIEA (12.0 eq) was added, and DMF (10 mL) was further added to the reaction column. The reaction column was blown with nitrogen. After the amino acid was dissolved, HATU (5.70 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.25 Coupling of Fmoc-Tyr(tBu)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Tyr(tBu)-OH (6.0 eq) was weighed and added to the above resin. DIEA (12.0 eq) was added, and DMF (10 mL) was further added to the reaction column. The reaction column was blown with nitrogen. After the amino acid was dissolved, HATU (5.70 eq) was added. The nitrogen was adjusted so that the resin was evenly blown.
3. The mixture was reacted in an environment of 25 °C for 2 h. The resin was tested with ninhydrin and showed blue.
4. The reaction solution was aspirated. The reaction column was washed with DMF 5 times (50 mL each time), for 1 min each time, and drained until no liquid flowed out.

**2.26 Coupling of Fmoc-Asp(OtBu)-OH**

1. 20% piperidine/DMF (50 mL) was added to the reaction column and the system was blown with nitrogen for 20

minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Asp(OtBu)-OH (6.0 eq) was weighed and added to the above resin. HOAT (6.0 eq) was added, and DMF (10 mL) was further added to the reaction column. The reaction column was blown with nitrogen. After the amino acid and HOAT were dissolved, DIC (6.0 eq) was added. The nitrogen was adjusted so that the resin was evenly blown.
3. The mixture was reacted in an environment of 25 °C for 1 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.27 Coupling of Fmoc-Ser(tBu)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Ser(tBu)-OH (6.0 eq) was weighed and added to the above resin. DIEA (12.0 eq) was added, and DMF (10 mL) was further added to the reaction column. The reaction column was blown with nitrogen. After the amino acid was dissolved, HATU (5.70 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.28 Coupling of Fmoc-Thr(tBu)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Thr(tBu)-OH (6.0 eq) was weighed and added to the above resin. DIEA (12.0 eq) was added, and DMF (10 mL) was further added to the reaction column. The reaction column was blown with nitrogen. After the amino acid was dissolved, HATU (5.70 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.29 Coupling of Fmoc-Phe-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Phe-OH (6.0 eq) was weighed and added to the above resin. DIEA (12.0 eq) was added, and DMF (10 mL) was further added to the reaction column. The reaction column was blown with nitrogen. After the amino acid was dissolved, HATU (5.70 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.30 Coupling of Fmoc-Thr(tBu)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Thr(tBu)-OH (6.0 eq) was weighed and added to the above resin. DIEA (12.0 eq) was added, and DMF (10 mL) was further added to the reaction column. The reaction column was blown with nitrogen. After the amino acid was dissolved, HATU (5.70 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.31 Coupling of Fmoc-Gly-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Gly-OH (3.0 eq) was weighed and added to the above resin. DIEA (6.00 eq) was added, and DMF (10 mL) was further added to the reaction column. The reaction column was blown with nitrogen. After the amino acid was dissolved, HATU (2.85 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.32 Coupling of Fmoc-Gln(Trt)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Gln(Trt)-OH (6.0 eq) was weighed and added to the above resin. HOBT (6.0 eq) was added, and DMF (10 mL) was further added to the reaction column. The reaction column was blown with nitrogen. After the amino acid and HOBT were dissolved, DIC (6.0 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

**2.33 Coupling of Fmoc-Aib-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. Fmoc-Aib-OH (3.0 eq) was weighed and added to the above resin. DIEA (6.0 eq) was added, and DMF (10 mL) was further added to the reaction column. The reaction column was blown with nitrogen. After the amino acid was

dissolved, HATU (2.85 eq) was added. The nitrogen was adjusted so that the resin was evenly blown.
3. The mixture was reacted in an environment of 25 °C overnight. The resin was tested with ninhydrin and was colorless and transparent.
4. The reaction solution was aspirated. The reaction column was washed with DMF 5 times (50 mL each time), for 1 min each time, and drained until no liquid flowed out.

**2.34 Coupling of Boc-His(Trt)-OH**

1. 20% piperidine/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with chloranil and showed blue.
2. Boc-His(Trt)-OH (6.0 eq) was weighed and added to the above resin. DIEA (12.0 eq) was added, and DMF (10 mL) was further added to the reaction column. The reaction column was blown with nitrogen. After the amino acid was dissolved, HATU (5.70 eq) was added. The nitrogen was adjusted so that the resin was evenly blown.
3. The mixture was reacted in an environment of 25 °C for 0.5 h. The resin was tested with chloranil and was colorless and transparent.
4. The reaction solution was aspirated. The reaction column was washed with DMF 5 times (50 mL each time), for 1 min each time, and drained until no liquid flowed out.

**2.35 Removal of Alloc**

1. $PhSiH_3$ (10.0 eq) and DCM (10 mL) were added to the reaction column and the system was blown with nitrogen. Then, $Pd(PPh_3)_4$ (0.1 eq) was added and the system was blown with nitrogen for 20 minutes. The mixture was reacted twice, and the reaction column was drained until no liquid flowed out.
2. The reaction column was washed with DMF 5 times (50 mL each time), for 1 min each time, and drained until no liquid flowed out.

**2.36 Coupling of Fmoc-Ida-OH**

1. Fmoc-Ida-OH (6.0 eq) was weighed and added to the above resin. DIEA (12.0 eq) was added, and DMF (10 mL) was further added to the reaction column. The reaction column was blown with nitrogen. After the amino acid was dissolved, HBTU (5.70 eq) was added. The nitrogen was adjusted so that the resin was evenly blown.
2. The mixture was reacted in an environment of 25 °C for 0.5 h. The resin was tested with ninhydrin and was colorless and transparent.
3. The reaction solution was aspirated. The reaction column was washed with DMF 5 times (50 mL each time), for 1 min each time, and drained until no liquid flowed out.

**2.37 Coupling of intermediate 1**

1. 10% DBU/DMF (50 mL) was added to the reaction column and the system was blown with nitrogen for 20 minutes. The reaction column was drained until no liquid flowed out. DMF (50 mL) was added to wash 5 times, for 1 min each time. The reaction column was drained until no liquid flowed out. The resin was tested with ninhydrin and showed blue.
2. **Intermediate 1** (1.50 eq) was weighed and added to the above resin. DIEA (3.00 eq) was added, and DMF (10 mL) was further added to the reaction column. The reaction column was blown with nitrogen. After the amino acid was dissolved, HBTU (1.45 eq) was added. The nitrogen was adjusted so that the resin was evenly blown.
3. The mixture was reacted in an environment of 25 °C for 0.5 h. The resin was tested with ninhydrin and was colorless and transparent.
4. The reaction solution was aspirated. The reaction column was washed with DMF 5 times (50 mL each time), for 1 min each time, and drained until no liquid flowed out.

### 2.38 Removal of Dde

1. 3% hydrazine hydrate/DMF (50 mL) was added to the reaction column and the system was blown with nitrogen for 15 minutes. The reaction column was drained. DMF (50 mL) was added to wash 5 times, for 1 min each time. The reaction column was drained until no liquid flowed out. The resin was tested with ninhydrin and showed blue.

### 2.39 Closing of amide ring

1. DIEA (3.0 eq) was added to the DMF solution of the above resin. Then, HATU (1.5 eq) dissolved in DMF was slowly added dropwise to the reaction column and the system was blown with nitrogen. The nitrogen was adjusted so that the resin was evenly blown.
2. The mixture was reacted in an environment of 25 °C for 0.5 h. The resin was tested with ninhydrin and was colorless and transparent.
3. The reaction solution was aspirated. The reaction column was washed with DMF 5 times (50 mL each time), for 1 min each time, and drained until no liquid flowed out.
4. MeOH (50 mL) was used to shrink the resin for 3 minutes each time. The reaction column was drained until no liquid flowed out. The resin was poured out and dried for later use.

### 3. Cleavage and drying of crude peptide

3.1. The cleavage solution was prepared according to the following volume: TFA/triisopropylsilane/$H_2O$/3-mercaptopropionic acid=90/2.5/2.5/5.

3.2 The dried peptide resin was added to the prepared cleavage solution. The mixture was shaken on a shaker for 2.5 hours and filtered. The filtrate was added to 10 times the volume of ice-cold isopropyl ether. The mixture was centrifuged, washed 5 times with isopropyl ether, and vacuum dried for 2 h to give a crude peptide. The crude peptide was separated and purified by preparative HPLC (purification step: mobile phase acetonitrile/water (40%/60%), 0.075% TFA; salt conversion step: mobile phase acetonitrile/water (20%/80%), 0.01% ammonium acetate) to give polypeptide WX001. The molecular weight of the polypeptide was confirmed by ESI-MS, with a calculated value of 4660.1 and a measured value of 4660.2.

## Example 2

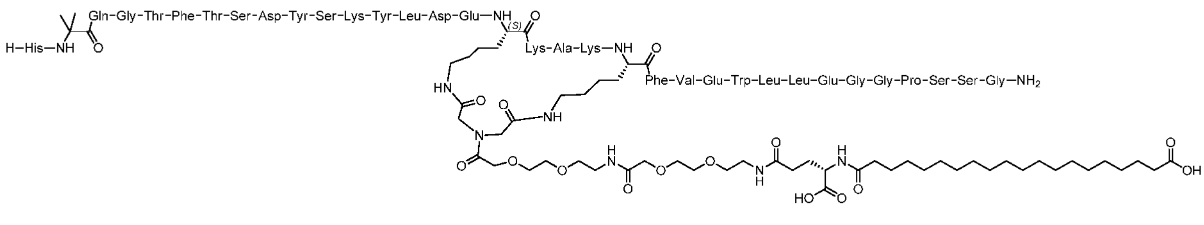

**WX002**

Polypeptide **WX002** was obtained by referring to the synthesis of **WX001.** The molecular weight of the polypeptide was confirmed by ESI-MS, with a calculated value of 4659.2 and a measured value of 4659.0.

## Example 3

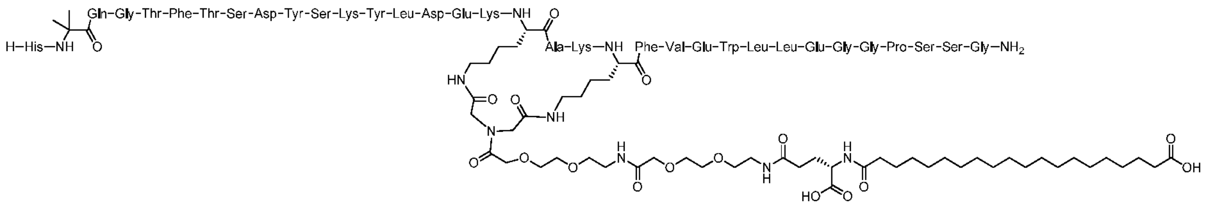

WX003

Polypeptide WX003 was obtained by referring to the synthesis of WX001. The molecular weight of the polypeptide was confirmed by ESI-MS, with a calculated value of 4659.2 and a measured value of 4659.3.

Example 4

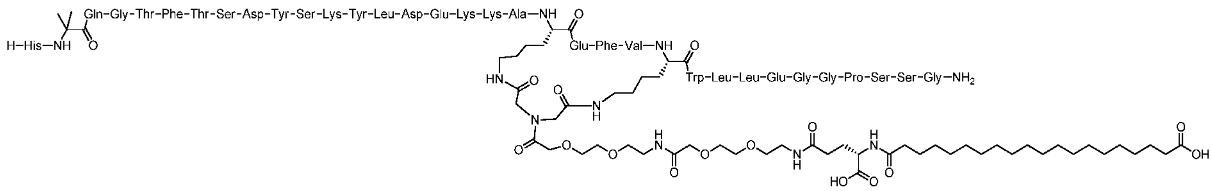

WX004

Polypeptide WX004 was obtained by referring to the synthesis of WX001. The molecular weight of the polypeptide was confirmed by ESI-MS, with a calculated value of 4659.2 and a measured value of 4659.6.

**Example 5**

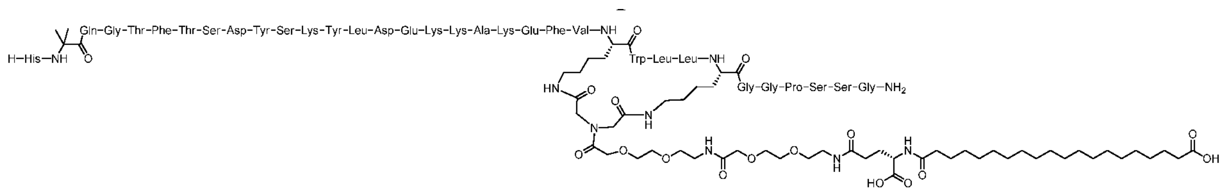

WX005

Polypeptide **WX005** was obtained by referring to the synthesis of **WX001.** The molecular weight of the polypeptide was confirmed by ESI-MS, with a calculated value of 4658.3 and a measured value of 4658.7.

**Biological assay data**

**Assay example 1: Assay of agonistic activity on GLP-1R/GIPR/GCGR *in vitro***

**A: Main materials:**

**(I) Cell line**

The cell lines were constructed by Shanghai WuXi AppTec. See Table 1 for details.

Table 1: Information of cell lines

| Target | Host cell | Cloning |
|---|---|---|
| GLP-1R | HEK293 | N/A |
| GCGR | HEK293 | N/A |
| GIPR | CHO | N/A |

**(II) Reagents and consumables**

See Table 2 for details.

Table 2: Information of reagents and consumables

| Name | Batch | Item number | Manufacturer |
|---|---|---|---|
| cAMP assay kit | 29F | 62AM4PEJ | Cisbio |
| 1M HEPES | 2120919 | 15630-106 | Invitrogen |
| Hanks' Balanced Salt Solution (HBSS) | 2185775 | 14025 | Invitrogen |
| Human serum albumin (HSA) | SLCF7301 | A1653-10G | Sigma |
| Casein | SLCC9458 | C4765-10mL | Sigma |
| 3-isobutyl-1-methylxanthine (IBMX) | STBF6061V | I5879-5G | Sigma |
| ECHO qualified 384-well plate | 0006433672 | PP-0200 | Labcyte |
| OptiPlate-3 84 | 8210-19481 | 6007299 | PerkinElmer |

**(III) Instrument**

See Table 3 for details.

Table 3: Information of instrument

| Name | Model | Manufacturer |
|---|---|---|
| EnVision | envision2014 | PerkinElmer |
| Vi-cell counter | Vi-CELL™ XR Cell Viability Analyzer | Beckman |
| Bravo | Bravo V11 | Agilent |
| ECHO | ECHO 555 | Labcyte |
| Centrifuge | Allegra™ 25R Centrifuge | Beckman |

**B. Method**

i) Assay materials

**The assay buffers are shown in Table 4.**

Table 4: Information of assay buffers

| **Reagent** | **Storage concentration** | **Volume** | **Final concentration** |
|---|---|---|---|
| Hanks' Balanced Salt Solution | 1x | 48.7 mL/ 44.7mL | ≈1x |
| HEPES buffer | 1mol/L | 250 μL | 5mmol/L |
| 5% casein solution (HEPES)/ 10% human serum albumin solution (HSA) | 5%/ 10% | 1000 μL/ 5000μL | 0.10%/ 1% |

(continued)

| Reagent | Storage concentration | Volume | Final concentration |
|---|---|---|---|
| 3-isobutyl-1-methylxanthine (IBMX) | 500mmol/L | 50 μL | 0.5mmol/L |

**Preparation of the assay reagent is shown in Table 5.**

Table 5: Information of the assay reagent preparation

| Reagent | Storage concentration | Volume | Final concentration |
|---|---|---|---|
| Cell lysates | 1x | 9.5 mL | ≈1x |
| D2-cAMP solution | 40x | 250 μL | 1x |
| cAMP-antibody solution | 40x | 250 μL | 1x |

**ii) Assay method**

a) Preparation of compound plate:

The compounds to be assayed were serially diluted 4-fold with Bravo to obtain 10 concentrations, with a starting concentration of 30 μM.

b) Transfer of compound:

1) 100 nL of the compound was transferred to an OptiPlate-384 plate using Echo.
2) The OptiPlate-384 plate was centrifuged at 1000 rpm for 5 seconds.

c) Preparation of cell suspension

1) A GLP-1R/GIPR/GCGR cell cryopreservation tube was quickly placed in warm water at 37 °C to thaw.
2) The cell suspension was transferred to a 15 mL centrifuge tube, and gently washed with 10 ml of HBSS.
3) The centrifuge tube was centrifuged at 1000 rpm at room temperature for 1 minute.
4) The supernatant was discarded.
5) The cells at the bottom were gently dispersed and gently washed with 10 mL of HBSS. The cells were centrifuged and sedimented, and finally resuspended with the assay buffer.
6) Vi-cell was used to measure the cell density and viability.
7) The concentration of GLP-1R/GIPR/GCGR cells was diluted to $2.0*10^5$/mL with the assay buffer.
8) 100 nL of the diluted cell suspension was transferred to the OptiPlate-384 plate.
9) The plate was incubated at room temperature for 30 minutes.

d) Addition of assay reagent:

1) 10 μL of 800nM gradient diluted cAMP standard was added into an empty well of the OptiPlate-384 plate.
2) 10μL of cAMP assay reagent was added.
3) The OptiPlate-384 plate was covered with a TopSeal-A film and incubated at room temperature for 60 minutes.

The TopSeal-A was peeled off and the plate was read on the EnVision.

**C Assay results**

The assay results are shown in Table 6.

Table 6: Assay results of in vitro agonistic activity on GLP-1R/GCGR/GIPR

| ID | Assay results of the activity | | | | | | | | |
|---|---|---|---|---|---|---|---|---|---|
| | Agonistic activity on GLP-1R | | | Agonistic activity on GCGR | | | Agonistic activity on GIPR | | |
| | $EC_{50}$ (nM) | Max Dose (nM) | Max Activity (%) | $EC_{50}$ (nM) | Max Dose (nM) | Max Activity (%) | $EC_{50}$ (nM) | Max Dose (nM) | Max Activit y (%) |
| WX001 | 0.02271 | 20 | 122.64 | 0.02669 | 100 | 98.93 | >20 | 20 | 2.26 |
| WX002 | 0.01692 | 20 | 106.52 | 0.1948 | 100 | 144.14 | >20 | 20 | 4.06 |
| WX003 | 0.05822 | 20 | 111.18 | 0.6244 | 100 | 86.01 | >20 | 20 | 1.57 |
| WX004 | 0.04459 | 20 | 85.77 | 0.05312 | 100 | 68.62 | >20 | 20 | 4.12 |
| WX005 | 0.1122 | 20 | 104.24 | 2.993 | 100 | 140.57 | >20 | 20 | 1.42 |

**Conclusion:** The compounds of the present disclosure have very strong agonistic activity on GLP-1R/GCGR, but have no agonistic activity on GIPR.

**Assay example 2: Drug efficacy study in DIO mice-in vivo drug efficacy evaluation**

**A. Purpose of the assay**

The weight loss effect of the assay compound in DIO mice was studied.

**B. Procedure of the assay**

1. After arriving at the facility, the DIO mice were raised in an animal breeding room with strictly controlled environmental conditions. The temperature in the breeding room was maintained at 20 to 24 °C, and the humidity was maintained at 30 to 70%. The temperature and humidity in the breeding room were monitored in real time using a thermo-hygrometer, and the temperature and humidity were recorded twice a day (once in the morning and once in the afternoon). The lighting in the animal breeding room was controlled by an electronic timed lighting system. The lights were on for 12 hours and off for 12 hours every day (turned on at 7:00 am and turned off at 19:00 pm). During the assay, the animals were raised in individual cages and toys were provided in each cage. During the assay, the animals had free access to food (growth/breeding feed for rats and mice) and water.
2. The animals in each group were subcutaneously injected with a solvent or an assay compound (10 nmol/kg), respectively. The administration time was 9:30 in the morning. The administration frequency was once every three days, and the administration cycle was 21 days.

**C. Assay results**

The assay results are shown in Table 7.

Table 7: Drug efficacy of the assay compound in DIO mice

| Compound number | Solvent | WX001 | WX004 |
|---|---|---|---|
| ΔBody weight % (comparison of the body weight after 21 days with the body weight on day 1) | 3.28% | -28.91% | -31.89% |

Conclusion: The compounds of the present disclosure exhibit excellent weight loss efficacy in DIO mice.

## Assay example 3. Assay of plasma protein binding (PPB)

### A. Purpose of the assay

The binding degree of the assay compound to human/mouse plasma albumin was studied.

### B. Procedure of the assay

1. Matrix preparation: on the day of the assay, plasma was thawed in cold water and centrifuged at 3220 rpm for 5 min to remove all clots. The pH of the resulting plasma was measured and adjusted to 7.4 ± 0.1 using 1% phosphoric acid or 1N sodium hydroxide as needed.

2. Dilution procedure for the assay compound: the assay compound was dissolved in dimethyl sulfoxide (DMSO) to prepare stock solutions with concentrations of 10 mM and 2 mM, respectively. A 40 μM working solution was prepared by diluting 2 μL of stock solution (2 mM) with 98 μL of DMSO. A 400 μM working solution of the control compound was prepared by diluting 10 μL of stock solution with 240 μL of DMSO. The working solution of the compound (5 μL) was mixed well with a blank matrix (995 μL) at a ratio of 1:200 to prepare a loading matrix.

3. Analysis steps

3.1 An equal volume of 30 μL of loading matrix (n=2) was transferred to a sample collection plate to prepare a time 0 (T0) sample for residue determination. The sample was immediately matched with the corresponding blank buffer to a final volume of 60 μL, and the volume ratio of plasma to buffer in each well was 1:1. Then, 60 μL of 4% $H_3PO_4$ in $H_2O$ and 480 μL of stop solution containing the internal standard were added to the T0 sample of the assay compound. They were then stored with other samples at 2-8 °C for further processing.

3.2 The remaining plasma samples were pre-incubated in a carbon dioxide incubator at 37 ± 1 °C for 30 min. Protein-free samples (F samples) and samples loaded with matrix (230 μL) were all transferred into polycarbonate tubes (n = 2) and ultracentrifuged at 37 °C and 155,000 × g (35,000 rpm) for 4 h.

3.3 To prepare T samples (assay samples), an additional matrix-containing sample was transferred to a separate 96-well plate (sample incubation plate) and incubated at 37 °C for 4 h.

3.4 At the end of centrifugation, 30 μL of protein-free samples and 30 μL of T samples were transferred from the second layer of the supernatant (below the top layer) to a new sample collection plate. Each sample was mixed with the corresponding blank buffer or matrix to a final volume of 60 μL with a matrix:buffer volume ratio of 1:1. 60 μL of 4% $H_3PO_4$ aqueous solution and 480 μL of stop solution (with internal standard) were added to all samples. The mixture was centrifuged at 4000 rpm for 20 min and 100 μL of supernatant from each sample was analyzed by LC-MS/MS.

### C. Assay results

The assay results are shown in Table 8:

Table 8: Assay results of PPB

| Compound number | WX001 | WX004 |
|---|---|---|
| PPB% unbound (human/mouse) | NA/NA | NA/NA |
| Note: NA indicates that the plasma protein binding is too high, and no free drug is detected at a normal plasma protein concentration. | | |

Conclusion: The compounds of the present disclosure have extremely high plasma protein binding.

## Assay example 4: Plasma stability assay (PLS)

### A. Purpose of the assay

Stability of the assay compound in normal mouse plasma was studied.

**B. Procedure of the assay**

1. Before the assay, the coagulated frozen plasma was thawed in a 37 °C water bath. The plasma was centrifuged at 4000 rpm for 5 minutes. If there were blood clots, the blood clots were removed. The pH value was adjusted to 7.4±0.1.
2. Preparation of assay compound solution: An assay compound was dissolved in DMSO to prepare a 100 μM solution.
3. 98 μL of blank control plasma was added to 2 μL of the assay compound solution (100 μM), so that the final concentration of the mixed solution reached 2 μM. The mixed solution was incubated in a 37 °C water bath.
4. 100 μL of $H_3PO_4$ solution and 800 μL of stop solution (a solution of 200 ng/mL tolbutamide and 200 ng/mL labetalol in 100% methanol) were added at each time point (0, 10, 30, 60 and 120 min), respectively, to precipitate proteins, and mixed thoroughly.
5. The sample was centrifuged at 4000 rpm for 20 min. 100 μL of supernatant from each well was taken for LC-MS/MS analysis.

**C. Assay results**

The assay results are shown in Table 9.

Table 9: Assay results of PLS

| **Compound number** | **WX001** |
|---|---|
| PLS (human/mouse) $T_{1/2}$ (min) | >289/>289 |

Conclusion: The compound of the present disclosure has excellent plasma stability.

**Assay example 5: Pharmacokinetic evaluation of the compound in mice**

**A. Purpose of the assay**

Pharmacokinetics of the compound in C57BL/6 mice were assayed.

**B. Procedure of the assay**

The pharmacokinetic characteristics of the compound in rodents after a subcutaneous injection were assayed using the standard protocol. In the assay, the candidate compound was formulated into a clear solution and given to mice by a single subcutaneous injection (SC, 0.048 mpk). The solvent of the subcutaneous injection was citrate buffer (20 mM, pH=7). Whole blood was collected, and plasma was prepared. Drug concentration was analyzed by LC-MS/MS method, and pharmacokinetic parameters were calculated using Phoenix WinNonlin software.

**C. Assay results**

The assay results are shown in Table 10.

Table 10: Pharmacokinetic assay results

| **Compound number** | **Maximum plasma concentration (nM)** | **$T_{max}$ (h)** | **Half-life $T_{1/2}$ (h)** | **Integral of the SC concentration $AUC_{0-72h}$(nM.hr)** |
|---|---|---|---|---|
| **WX001** | 46 | 8 | 21 | 1809 |
| **WX004** | 36 | 12 | 22 | 1161 |

Conclusion: The compounds of the present disclosure have excellent pharmacokinetic properties in mice.

**Assay example 6: Pharmacokinetic evaluation of the compound in cynomolgus monkeys**

**A. Purpose of the assay**

Pharmacokinetics of the compound in cynomolgus monkeys were assayed.

**B. Procedure of the assay**

The pharmacokinetic characteristics of the compound in mammals after a subcutaneous injection were assayed using the standard protocol. In the assay, the candidate compound was formulated into a clear solution and given to cynomolgus monkeys by a single subcutaneous injection (SC, 0.02 mpk). The solvent of the subcutaneous injection was citrate buffer (20 mM, pH=7). Whole blood was collected, and plasma was prepared. Drug concentration was analyzed by LC-MS/MS method, and pharmacokinetic parameters were calculated using Phoenix WinNonlin software.

**C. Assay results**

The assay results are shown in Table 11.

Table 11: Pharmacokinetic assay results

| Compound number | Maximum plasma concentration (nM) | $T_{max}$ (h) | Half-life $T_{1/2}$ (h) | Integral of the SC concentration $AUC_{0-240h}$(nM.hr) |
|---|---|---|---|---|
| **WX001** | 43 | 24 | 93 | 5510 |
| **WX004** | 48 | 32 | 67 | 5719 |

Conclusion: The compounds of the present disclosure have excellent pharmacokinetic properties in monkeys.

**Assay example 7: Verification of in vivo drug efficacy in STZ-NASH mouse model**

**A. Purpose of the assay**

Drug efficacy of the assay substance in the NASH model induced by streptozotocin (STZ) + high-fat diet (HFD) in C57BL/6 mice was verified.

**B. Procedure of the assay**

Modeling method: Newborn mice were subcutaneously injected with STZ (200 μg/mouse) within 48 hours after birth. After 4 weeks of breastfeeding, animals with a fasting blood glucose level of > 12 mmol/L were selected and subjected to a HFD feeding for 6 consecutive weeks to finally establish the NASH model. Another 8 animals were selected without STZ injection and HFD feeding (normal control group).
Administration scheme: Administration was started after one week of HFD feeding, and the first day of administration was set as Day 1. Then, the mice were subcutaneously injected every two days for 5 consecutive weeks.
Detection at the endpoint of the assay: Pathological hematoxylin-eosin staining and picro sirius red staining were performed.

**C. Assay results**

The assay results are shown in Table 13.

Table 13 Animal pathological indicators of the STZ-NASH model at endpoint

| | Solvent control group of the model | Solvent control group of the model | WX001 (20nmol/kg) |
|---|---|---|---|
| Steatosis score | 0 | 2.54 | 1.0 |
| Inflammation score | 0 | 1.67 | 1.63 |
| Ballooning score | 0 | 1.29 | 0 |

(continued)

| | Solvent control group of the model | Solvent control group of the model | WX001 (20nmol/kg) |
|---|---|---|---|
| NAS score | 0 | 5.5 | 2.63 |
| Fibrosis (%) | 0.71 | 1.11 | 1.21 |

Conclusion: The compound of the present disclosure can significantly improve the NAS score in the STZ-NASH mouse model.

## Claims

1. A polypeptide represented by a sequence shown below,

His-Aib-Gln-Gly-Thr-Phe-Thr-Ser-Asp-Tyr-Ser-Lys-Tyr-Leu-Asp-Glu-$^{1}$Lys-Lys-Ala-$^{1}$Lys-Glu-Phe-Val-Glu-Trp-Leu-Leu-Glu-Gly-Gly-Pro-Ser-Ser-$X_2$ (1-1)

His-Aib-Gln-Gly-Thr-Phe-Thr-Ser-Asp-Tyr-Ser-Lys-Tyr-Leu-Asp-Glu-$^{1}$Lys-Lys-Ala-Lys-$^{1}$Lys-Phe-Val-Glu-Trp-Leu-Leu-Glu-Gly-Gly-Pro-Ser-Ser-$X_2$ (I-2)

His-Aib-Gln-Gly-Thr-Phe-Thr-Ser-Asp-Tyr-Ser-Lys-Tyr-Leu-Asp-Glu-Lys-$^{1}$Lys-Ala-Lys-$^{1}$Lys-Phe-Val-Glu-Trp-Leu-Leu-Glu-Gly-Gly-Pro-Ser-Ser-$X_2$ (I-3)

His-Aib-Gln-Gly-Thr-Phe-Thr-Ser-Asp-Tyr-Ser-Lys-Tyr-Leu-Asp-Glu-Lys-Lys-Ala-$^{1}$Lys-Glu-Phe-Val-$^{1}$Lys-Trp-Leu-Leu-Glu-Gly-Gly-Pro-Ser-Ser-$X_2$ (I-4)

His-Aib-Gln-Gly-Thr-Phe-Thr-Ser-Asp-Tyr-Ser-Lys-Tyr-Leu-Asp-Glu-Lys-Lys-Ala-Lys-Glu-Phe-Val-$^{1}$Lys-Trp-Leu-Leu-$^{1}$Lys-Gly-Gly-Pro-Ser-Ser-$X_2$ (I-5)

wherein

the structure of Aib is

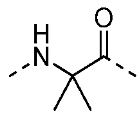
;

$X_2$ is selected from

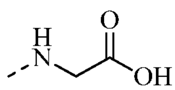
and
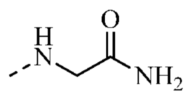
;

$^{1}$Lys represents a modified lysine, and the modification is that the amino groups on the two lysine side chains are connected to

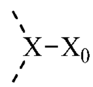
;

X is selected from connected to $X_0$;

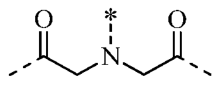 and 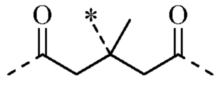 ,

wherein "*" indicates the position
$X_0$ is

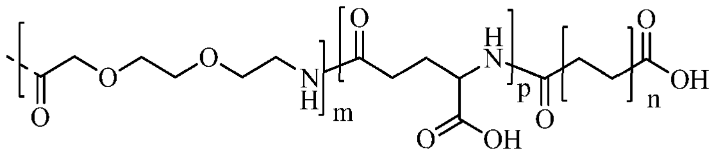
;

m is selected from 2 and 3;
n is selected from 8, 9 and 10;
p is selected from 1 and 2.

**2.** The polypeptide according to claim 1, wherein m is 2.

**3.** The polypeptide according to claim 1, wherein n is 9.

**4.** The polypeptide according to claim 1, wherein p is 1.

**5.** The polypeptide according to claim 1, wherein $X_2$ is

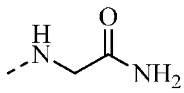
.

**6.** The polypeptide according to claim 1, wherein $X_0$ is

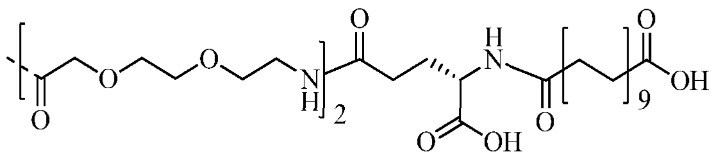
.

**7.** The polypeptide according to claim 1, wherein the structural unit

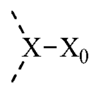

is

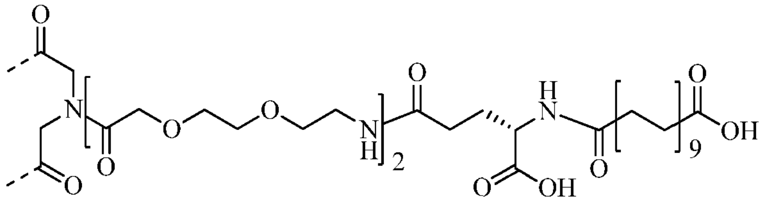
.

**8.** A polypeptide represented by the following formulas,

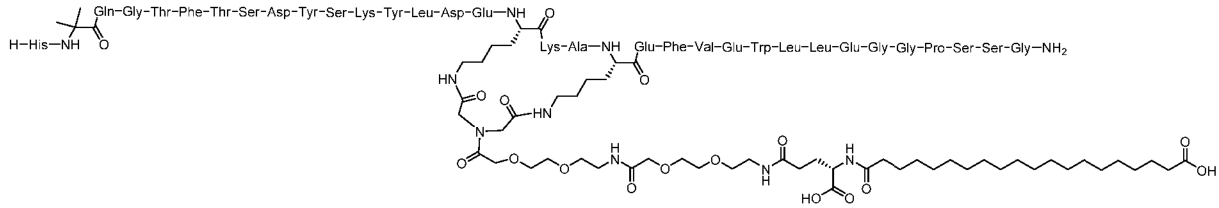

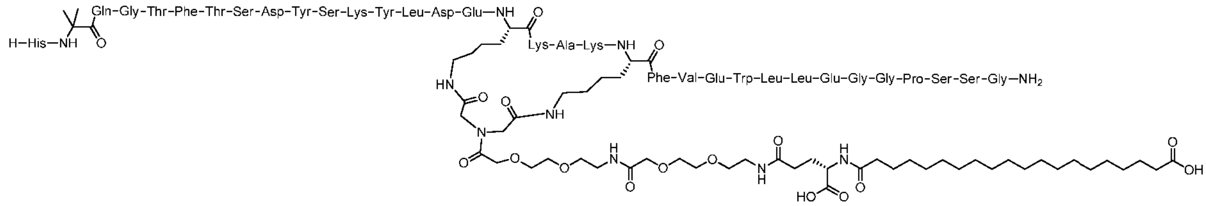

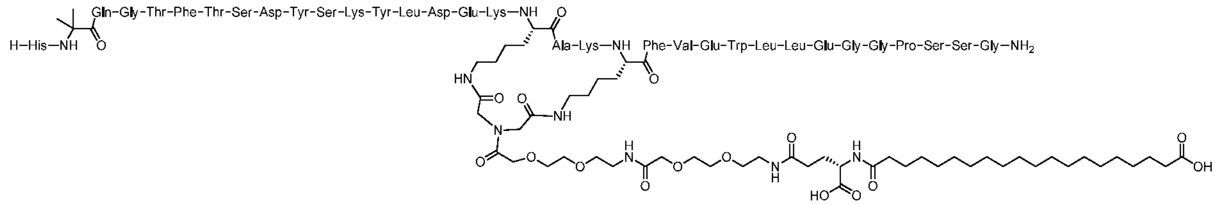

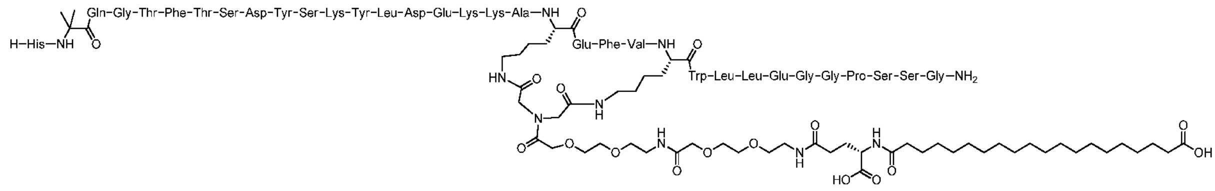

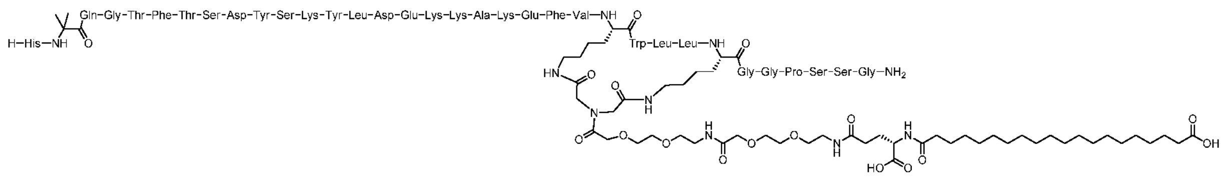

.

**9.** Use of the polypeptide compound according to any one of claims 1 to 8 in the manufacture of a medicament for the treatment of a GLP-1R/GCGR-related disease.

**10.** The use according to claim 9, wherein the GLP-1R/GCGR-related disease is obesity or nonalcoholic steatohepatitis.

## INTERNATIONAL SEARCH REPORT

International application No.
**PCT/CN2022/130781**

**A. CLASSIFICATION OF SUBJECT MATTER**

C07K 14/605(2006.01)i；A61K 38/26(2006.01)i；A61P 1/00(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNTXT, WPABSC, WPABS, ENTXTC, ENTXT, DPWI, VEN, CNKI, Web of Science, 百度学术, BAIDU SCHOLAR, HimmPat, incoPat, 中国生物序列检索系统, Chinese Patent Biological Sequence Retrieval System, NCBI, EBI, STNext: 申请人, 发明人, 胰高血糖素样肽-1, GLP-1, glucagon like peptide-1, 订合肽, 订书肽, 装订肽, staple peptide, 序列1-5

**C. DOCUMENTS CONSIDERED TO BE RELEVANT**

| Category* | Citation of document, with indication, where appropriate, of the relevant passages | Relevant to claim No. |
|---|---|---|
| Y | CN 107735100 A (ELI LILLY AND COMPANY) 23 February 2018 (2018-02-23)<br>claims 1-44, and description, paragraphs 11-76 | 1-10 |
| Y | CN 107921098 A (THE CALIFORNIA INSTITUTE FOR BIOMEDICAL RESEARCH) 17 April 2018 (2018-04-17)<br>claims 1-41, and description, paragraphs 5-8 and 35-63 | 1-10 |
| Y | CN 106456589 A (THE CALIFORNIA INSTITUTE FOR BIOMEDICAL RESEARCH et al.) 22 February 2017 (2017-02-22)<br>claims 1-71 | 1-10 |
| Y | CN 109745547 A (SHENZHEN TURIER BIOTECH DEVELOPMENT CO., LTD.) 14 May 2019 (2019-05-14)<br>claims 1-19 | 1-10 |
| Y | CN 102918055 A (NOVO NORDISK A/S) 06 February 2013 (2013-02-06)<br>claims 1-18 | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 January 2023** | **20 January 2023** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/130781**

**C. DOCUMENTS CONSIDERED TO BE RELEVANT**

| Category* | Citation of document, with indication, where appropriate, of the relevant passages | Relevant to claim No. |
|---|---|---|
| A | JI, L et al. "IBI362 (LY3305677), a weekly-dose GLP-1 and glucagon receptor dual agonist, in Chinese adults with overweight or obesity: A randomised, placebo-controlled, multiple ascending dose phase 1b study." *ECLINICALMEDICINE.*, Vol. 39, 13 August 2021 (2021-08-13), article number 101088, pages 1-8 | 1-10 |

Form PCT/ISA/210 (second sheet) (January 2015)

**INTERNATIONAL SEARCH REPORT**

International application No.
**PCT/CN2022/130781**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   [1] The actually submitted sequence listing is an XML file in Standard ST.26.

**INTERNATIONAL SEARCH REPORT**
**Information on patent family members**

International application No.
**PCT/CN2022/130781**

| Patent document cited in search report | Publication date (day/month/year) | Patent family member(s) | Publication date (day/month/year) |
|---|---|---|---|
| CN 107735100 A | 23 February 2018 | KR 20190119676 A | 22 October 2019 |
| | | MA 46219 A | 25 April 2018 |
| | | MD 3310371 T2 | 31 December 2020 |
| | | PH 12017502359 A1 | 25 June 2018 |
| | | MA 54407 A | 13 October 2021 |
| | | HU E052684 T2 | 28 May 2021 |
| | | CA 2987489 A1 | 29 December 2016 |
| | | IL 294099 A | 01 August 2022 |
| | | CO 2018000078 A2 | 28 March 2018 |
| | | AU 2016283984 A1 | 23 November 2017 |
| | | HK 1245669 A1 | 31 August 2018 |
| | | JP 2020007338 A | 16 January 2020 |
| | | LT 3310371 T | 28 December 2020 |
| | | EC SP17084280 A | 28 February 2018 |
| | | RS 61107 B1 | 31 December 2020 |
| | | UA 122692 C2 | 28 December 2020 |
| | | JP 2022031787 A | 22 February 2022 |
| | | AR 104932 A1 | 23 August 2017 |
| | | SV 2017005594 A | 24 April 2018 |
| | | WO 2016209707 A1 | 29 December 2016 |
| | | PE 20180523 A1 | 14 March 2018 |
| | | TW 201716431 A | 16 May 2017 |
| | | HR P20201881 T1 | 22 January 2021 |
| | | TW 202118775 A | 16 May 2021 |
| | | CR 20170534 A | 27 February 2018 |
| | | BR 112017024684 A2 | 11 September 2018 |
| | | US 2016368960 A1 | 22 December 2016 |
| | | EA 201792562 A1 | 31 May 2018 |
| | | JO 3686 B1 | 27 August 2020 |
| | | DO P2017000303 A | 15 January 2018 |
| | | DK 3310371 T3 | 12 October 2020 |
| | | MY 190210 A | 05 April 2022 |
| | | NZ 737050 A | 30 August 2019 |
| | | PL 3310371 T3 | 08 March 2021 |
| | | CN 113956348 A | 21 January 2022 |
| | | TW 201906857 A | 16 February 2019 |
| | | IL 285231 A | 30 September 2021 |
| | | PT 3310371 T | 15 December 2020 |
| | | SI 3310371 T1 | 30 November 2020 |
| | | KR 20180004276 A | 10 January 2018 |
| | | TN 2017000531 A1 | 12 April 2019 |
| | | EP 3785724 A1 | 03 March 2021 |
| | | JP 2018521043 A | 02 August 2018 |
| | | MX 2017016198 A | 01 March 2018 |
| | | EP 3310371 A1 | 25 April 2018 |
| | | ES 2833458 T3 | 15 June 2021 |
| | | IL 255723 A | 31 January 2018 |
| | | CL 2017003185 A1 | 01 June 2018 |
| CN 107921098 A | 17 April 2018 | US 2022072104 A1 | 10 March 2022 |
| | | US 2019000928 A1 | 03 January 2019 |

Form PCT/ISA/210 (patent family annex) (January 2015)

**INTERNATIONAL SEARCH REPORT**
**Information on patent family members**

International application No.
**PCT/CN2022/130781**

| Patent document cited in search report | | | Publication date (day/month/year) | Patent family member(s) | | | Publication date (day/month/year) |
|---|---|---|---|---|---|---|---|
| | | | | WO | 2016205488 | A1 | 22 December 2016 |
| | | | | EP | 3310376 | A1 | 25 April 2018 |
| CN | 106456589 | A | 22 February 2017 | EP | 3082797 | A1 | 26 October 2016 |
| | | | | JP | 2017502024 | A | 19 January 2017 |
| | | | | AU | 2014364589 | A1 | 07 July 2016 |
| | | | | US | 2022000981 | A1 | 06 January 2022 |
| | | | | CA | 2933701 | A1 | 25 June 2015 |
| | | | | KR | 20160093074 | A | 05 August 2016 |
| | | | | US | 2018344812 | A1 | 06 December 2018 |
| | | | | WO | 2015095406 | A1 | 25 June 2015 |
| | | | | US | 2016317623 | A1 | 03 November 2016 |
| CN | 109745547 | A | 14 May 2019 | EP | 3708577 | A1 | 16 September 2020 |
| | | | | WO | 2019085772 | A1 | 09 May 2019 |
| | | | | CN | 111278853 | A | 12 June 2020 |
| | | | | US | 2021187074 | A1 | 24 June 2021 |
| | | | | CN | 114920818 | A | 19 August 2022 |
| | | | | CN | 109745549 | A | 14 May 2019 |
| CN | 102918055 | A | 06 February 2013 | JP | 2016183192 | A | 20 October 2016 |
| | | | | WO | 2011117415 | A1 | 29 September 2011 |
| | | | | JP | 2013523618 | A | 17 June 2013 |
| | | | | BR | 112012024379 | A2 | 10 January 2017 |
| | | | | WO | 2011117416 | A1 | 29 September 2011 |
| | | | | CA | 2792663 | A1 | 29 September 2011 |
| | | | | US | 2017190757 | A1 | 06 July 2017 |
| | | | | US | 2013035285 | A1 | 07 February 2013 |
| | | | | US | 2016002311 | A1 | 07 January 2016 |
| | | | | US | 2016271263 | A1 | 22 September 2016 |
| | | | | EP | 2552950 | A1 | 06 February 2013 |
| | | | | KR | 20130018410 | A | 21 February 2013 |
| | | | | ZA | 201206838 | B | 26 June 2013 |
| | | | | JP | 2013523619 | A | 17 June 2013 |
| | | | | EP | 2552951 | A1 | 06 February 2013 |
| | | | | IN | 8483CHENP2012 | A | 14 February 2014 |
| | | | | MX | 2012010881 | A | 06 November 2012 |
| | | | | CN | 102918056 | A | 06 February 2013 |
| | | | | US | 2017051034 | A1 | 23 February 2017 |
| | | | | US | 2015274801 | A1 | 01 October 2015 |
| | | | | RU | 2012144289 | A | 10 May 2014 |
| | | | | US | 2018016319 | A1 | 18 January 2018 |
| | | | | AU | 2011231503 | A1 | 27 September 2012 |
| | | | | US | 2013143798 | A1 | 06 June 2013 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2021114005918
- CN 2022106482638